# EUROPÄISCHE PATENTSCHRIFT

(11) **EP 1 315 473 B1**
(45) Veröffentlichungstag und Bekanntmachung des Hinweises auf die Patenterteilung: **29.06.2005**
(21) Anmeldenummer: 01976062.8
(22) Anmeldetag: 08.08.2001
(51) Int. Cl.: A61K 7/16

(54) **VERWENDUNG VON ECTOIN ODER ECTOIN-DERIVATEN ZUR MUNDPFLEGE**
USE OF ECTOINE OR ECTOINE DERIVATIVES FOR ORAL CARE
UTILISATION D'ECTOINE OU DE DERIVES D'ECTOINE POUR L'HYGIENE BUCCALE

(30) Priorität: 07.09.2000 DE 10044327
(43) Veröffentlichungstag der Anmeldung: 04.06.2003
(73) Patentinhaber: MERCK PATENT GmbH, 64293 Darmstadt (DE)
(72) Erfinder: BÜNGER, Joachim, 64823 Gross-Umstadt (DE); DRILLER, Hansjürgen, 64823 Gross-Umstadt (DE); DEN HOLLANDER, Olaf, NL-3066 HH Rotterdam (NL)
(86) Internationale Anmeldenummer: PCT/EP2001/009171
(87) Internationale Veröffentlichungsnummer: WO 2002/019978

(56) Entgegenhaltungen:
- WO-A-01/72263
- DE-A- 10 014 632
- US-A- 5 316 758
- US-A- 5 695 745
- J. BÜNGER: "Neue Wirkstoffklasse schützt und pflegt die Haut" PARFÜMERIE UND KOSMETIK, Bd. 79, Nr. 11, 1998, Seiten 32-34, XP000852629

## Beschreibung

Die Erfindung betrifft Zubereitungen zur Mundpflege.

Die Mundhöhle des Menschen wird von einer großen Anzahl kommensalisch lebender Bakterien, der residenten Mikroflora, besiedelt, welche an die speziellen Bedingungen im Mundraum angepasst sind. Die Mikroflora der Mundhöhle schützt vor der Kolonisierung durch pathogene Keime und ist für die Geruchsbildung, also den Mundgeruch, verantwortlich. Die Bakterien nutzen das Nahrungsangebot in der Mundhöhle und bilden beim Abbau der Nährstoffe riechende Substanzen wie beispielsweise kurzkettige Fettsäuren. Die mikrobielle Besiedlung und Ablagerung von Stoffwechselprodukten auf den Zähnen begünstigt die Plaque-Bildung. Die mikrobiellen Abbauprodukte aus Kohlenhydraten aus der Nahrung führen zu einer Reduktion des pH-Wertes und unterstützen die Karies-Bildung. Infolge der Säurebildung der Plaque-Bakterien kommt es zu einer Mikroentkalkung der Zähne. Die Bakterien sollten durch regelmäßige Plaqueentfernung und Beschränkung der häufigen Zuckeraufnahme bzw. Unterstützung der Wiederverkalkung durch häufige Fluoridierung geschützt werden.

In der älteren Internationalen Patentanmeldung WO 01/72263 werden eine Zahngelzusammensetzung und ein Mundwasser-Konzentrat beschrieben, die je 1 Gew.-% Ectoin enthalten.

Es bestand daher die Aufgabe, Zubereitungen zur Verfügung zu stellen, die zur Mundpflege geeignet sind.

Überraschend wurde nun gefunden, dass diese Aufgabe durch die Verwendung von einer oder mehreren Verbindungen ausgewählt aus den Verbindungen der Formeln Ia und Ib 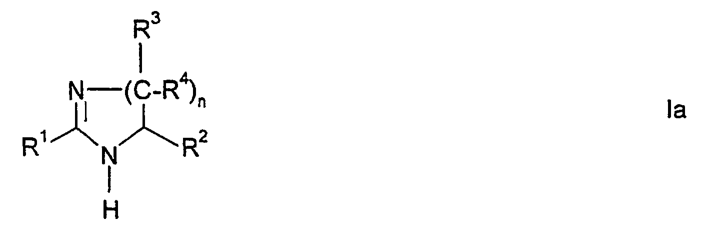 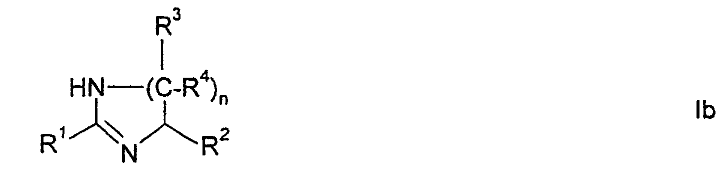 den physiologisch verträglichen Salzen der Verbindungen der Formeln Ia und Ib, und den stereoisomeren Formen der Verbindungen der Formeln Ia und Ib, wobei
- R¹: H oder Alkyl,
- R²: H, COOH, COO-Alkyl oder CO-NH-R⁵,
- R³ und R⁴: jeweils unabhängig voneinander H oder OH,
- n: 1, 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten,
in Zubereitungen gelöst wird.

Ectoin schützt die für eine intakte Hautbarriere wichtige Mikroflora der Haut und Schleimhaut gegen Stress durch Austrocknung, Radikale, Tenside und hohe Ionenkonzentration. Das Ectoin/Hydroxyectoin reagiert nicht mit dem Zellstoffwechsel.

Ectoine besitzen die Eigenschaft, Zellen, Proteine, Enzyme, DNA und Biomembranen vor Entzug von Wassermolekülen aus der Hydrathülle zu schützen und die räumliche Struktur der Biopolymere zu stabilisieren.

Die Stabilisierung der residenten Mundflora durch Ectoin oder seine Derivate ist eine wichtige Voraussetzung für das Gleichgewicht des Mikromilieus der Mundschleimhaut und die Ausbildung einer intakten Mundhöhlenflora. Durch die Stabilisierung der residenten Mundflora können sich transiente, schädigende Bakterien schlechter ansiedeln.

Ectoin schützt die Mundschleimhaut gegen Austrocknung, Tenside und andere Chemikalien. Eine Besiedlung der Mundschleimhaut mit Pathogenen wie z. B. dem Eitererreger Staphylococcus aureus wird durch die Stabilisierung der residenten Flora erschwert oder verhindert. Der Moisturizer-Effekt erschwert die Besiedlung durch Staphylococcus aureus zusätzlich, da diese Bakterien vor allem an Kollagen sowie Fibronektin in der geschädigten und trockenen Haut binden.

Der Gegenstand der vorliegenden Erfindung betrifft die Verwendung von einer oder mehreren Verbindungen ausgewählt aus den Verbindungen der Formeln Ia und Ib 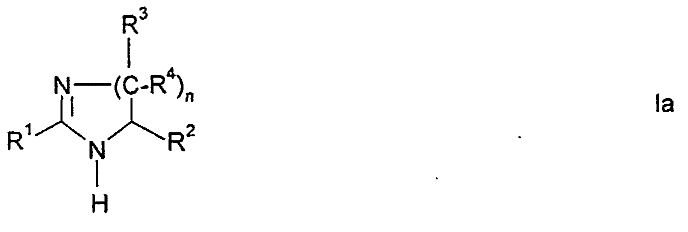 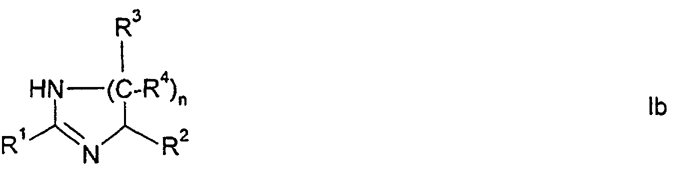 den physiologisch verträglichen Salzen der Verbindungen der Formeln Ia und Ib, und den stereoisomeren Formen der Verbindungen der Formeln Ia und Ib, wobei
- R¹: H oder Alkyl,
- R²: H, COOH, COO-Alkyl oder CO-NH-R⁵,
- R³ und R⁴: jeweils unabhängig voneinander H oder OH,
- n: 1, 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten, in einer Zubereitung zur Mundpflege.

Die Zubereitungen zur Mundpflege umfassen im Rahmen der vorliegenden Erfindung sowohl Mund- als auch Zahnpflegemittel.

In einer bevorzugten Ausführungsform betrifft die vorliegende Erfindung die Verwendung einer oder mehrerer Verbindungen ausgewählt aus den obengenannten Verbindungen der Formeln Ia und Ib, den physiologisch verträglichen Salzen der Verbindungen der Formeln Ia und Ib, und den stereoisomeren Formen der Verbindungen der Formeln Ia und Ib in Zubereitungen zum Schutz und zur Pflege der residenten Mundflora.

In einer weiteren bevorzugten Ausführungsform betrifft die vorliegende Erfindung die Verwendung einer oder mehrerer Verbindungen ausgewählt aus den obengenannten Verbindungen der Formeln Ia und Ib, den physiologisch verträglichen Salzen der Verbindungen der Formeln Ia und Ib, und den stereoisomeren Formen der Verbindungen der Formeln Ia und Ib in Zubereitungen zum prophylaktischen Schutz der Zähne gegen eine Schädigung des Zahnschmelzes.

In einer weiteren bevorzugten Ausführungsform betrifft die vorliegende Erfindung die Verwendung einer oder mehrerer Verbindungen ausgewählt aus den obengenannten Verbindungen der Formeln Ia und Ib, den physiologisch verträglichen Salzen der Verbindungen der Formeln Ia und Ib, und den stereoisomeren Formen der Verbindungen der Formeln Ia und Ib in Zubereitungen zum prophylaktischen Schutz der Mund- und Rachenschleimhaut.

Die Zubereitungen enthaltend eine oder mehrere Verbindungen ausgewählt aus den obengenannten Verbindungen der Formeln Ia und Ib, den physiologisch verträglichen Salzen der Verbindungen der Formeln Ia und Ib, und den stereoisomeren Formen der Verbindungen der Formeln Ia und Ib werden prophylaktisch angewendet.

Im Rahmen der vorliegenden Erfindung werden alle vor- und nachstehenden Verbindungen ausgewählt aus den Verbindungen der Formeln Ia und Ib, den physiologisch verträglichen Salzen der Verbindungen der Formeln Ia und Ib, und den stereoisomeren Formen der Verbindungen der Formeln Ia und Ib als "Ectoin oder Ectoin-Derivate" bezeichnet.

Bei Ectoin und den Ectoin-Derivaten handelt es sich um niedermolekulare, cyclische Aminosäurederivate, die aus verschiedenen halophilen Mikroorganismen gewonnen werden können. Sowohl Ectoin als auch Hydroxyectoin besitzen den Vorteil, dass sie nicht mit dem Zellstoffwechsel reagieren.

Die Verbindungen, ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib können in den Zubereitungen als optische Isomere, Diastereomere, Racemate, Zwitterionen, Kationen oder als Gemisch derselben vorliegen. Unter den Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib, sind diejenigen Verbindungen bevorzugt, worin R¹ H oder CH₃, R² H oder COOH, R³ und R⁴ jeweils unabhängig voneinander H oder OH und n 2 bedeuten. Unter den Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib sind die Verbindungen (S)-1,4,5,6-Tetrahydro-2-methyl-4pyrimidincarbonsäure (Ectoin) und (S,S)-1,4,5,6-Tetrahydro-5-hydroxy-2-methyl-4-pyrimidincarbonsäure (Hydroxyectoin) insbesondere bevorzugt.

Unter dem Begriff "Aminosäure" werden die stereoisomeren Formen, z. B. D- und L-Formen, folgender Verbindungen verstanden: Alanin, β-Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin, γ-Aminobutyrat, Nε-Acetyllysin, Nδ-Acetylornithin, Nγ-Acetyldiaminobutyrat und Nα-Acetyldiaminobutyrat. L-Aminosäuren sind bevorzugt.

Aminosäurereste leiten sich von den entsprechenden Aminosäuren ab.

Die Reste folgender Aminosäuren sind bevorzugt: Alanin, β-Alanin, Asparagin, Asparaginsäure, Glutamin, Glutaminsäure, Glycin, Serin, Threonin, Valin, γ-Aminobutyrat, Nε-Acetyllysin, Nδ-Acetylornithin, Nγ-Acetyldiaminobutyrat und Nα-Acetyldiaminobutyrat.

Die Di- und Tripeptidreste sind ihrer chemischen Natur nach Säureamide und zerfallen bei der Hydrolyse in 2 oder 3 Aminosäuren. Die Aminosäuren in den Di- und Tripeptidresten sind durch Amidbindungen miteinander verbunden. Bevorzugte Di- und Tripetidreste sind aus den bevorzugten Aminosäuren aufgebaut.

Die Alkylgruppen umfassen die Methylgruppe CH₃, die Ethylgruppe C₂H₅, die Propylgruppen CH₂CH₂CH₃ und CH(CH₃)₂ sowie die Butylgruppen CH₂CH₂CH₂CH₃, H₃CCHCH₂CH₃, CH₂CH(CH₃)₂ und C(CH₃)₃. Die bevorzugte Alkylgruppe ist die Methylgruppe.

Bevorzugte physiologisch verträgliche Salze der Verbindungen der Formeln Ia und Ib sind beispielsweise Alkali-, Erdalkali- oder Ammoniumsalze, wie Na-, K-, Mg- oder Ca-Salze, sowie Salze abgeleitet von den organischen Basen Triethylamin oder Tris-(2-hydroxy-ethyl)-amin. Weitere bevorzugte physiologisch verträgliche Salze der Verbindungen der Formeln Ia und Ib ergeben sich durch Umsetzung mit anorganischen Säuren wie Salzsäure, Schwefelsäure und Phosphorsäure oder mit organischen Carbon- oder Sulfonsäuren wie Essigsäure, Citronensäure, Benzoesäure, Maleinsäure, Fumarsäure, Weinsäure und p-Toluolsulfonsäure.

Verbindungen der Formeln Ia und Ib, in denen basische und saure Gruppen wie Carboxyl- oder Aminogruppen in gleicher Zahl vorliegen, bilden innere Salze.

Die Herstellung der Verbindungen der Formel Ia und Ib ist in der Literatur beschrieben (DE 43 42 550). (S)-1,4,5,6-Tetrahydro-2-methyl-4-pyrimidincarbonsäure oder (S,S)-1,4,5,6-Tetrahydro-5-hydroxy-2-methyl-4-pyrimidincarbonsäure können auch mikrobiologisch gewonnen werden (Severin et al., J. Gen. Microb.138 (1992) 1629-1638).

In einer bevorzugten Ausführungsform enthält die Zubereitung ein oder mehrere Antioxidantien. In den Zubereitungen können die aus der Fachliteratur bekannten Antioxidantien enthalten sein, z.B. Flavonoide, Coumaranone, Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat) sowie Koniferylbenzoat des Benzoeharzes, Rutinsäure und deren Derivate, α-Glycosylrutin, Ferulasäure, Furfurylidenglucitol, Carnosin, Butylhydroxytoluol (BHT), Butylhydroxyanisol, Nordohydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

In einer besonders bevorzugten Ausführungsform der Erfindung enthält die Zubereitung eine oder mehrere Verbindungen ausgewählt aus Flavonoiden und/oder Coumaranonen.

In einer weiteren besonders bevorzugten Ausführungsform der Erfindung enthält die Zubereitung als Antioxidans eine der oben genannten Mischungen enthaltend Lecithin, L-(+)-Ascorbylpalmitat und Zitronensäure (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Weiterhin besonders bevorzugt sind Ausführungsformen enthaltend eine dieser Mischungen und Flavonoide.

Als Flavonoide werden die Glykoside von Flavanonen, Flavonen, 3-Hydroxyflavonen (= Flavonolen), Auronen, Isoflavonen und Rotenoiden aufgefasst [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavanone sind durch folgende Grundstruktur gekennzeichnet:

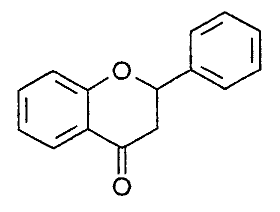

Die Flavone sind durch folgende Grundstruktur gekennzeichnet:

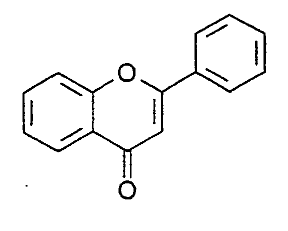

Die 3-Hydroxyflavone (Flavonole) sind durch folgende Grundstruktur gekennzeichnet:

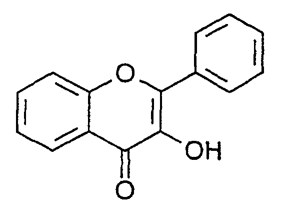

Die Isoflavone sind durch folgende Grundstruktur gekennzeichnet:

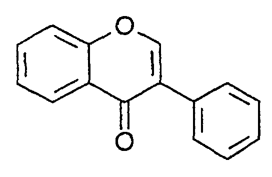

Die Aurone sind durch folgende Grundstruktur gekennzeichnet:

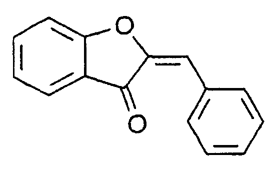

Die Coumaranone sind durch folgende Grundstruktur gekennzeichnet:

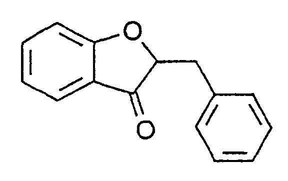

Vorzugsweise sind die Flavonoide und Coumaranone ausgewählt aus den Verbindungen der Formel (I): 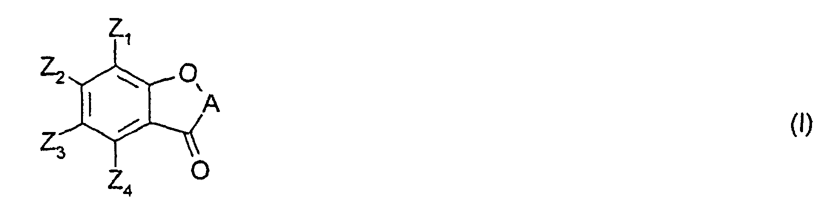 worin
- Z₁ bis Z₄: jeweils unabhängig voneinander H, OH, Alkoxy, Hydroxyalkoxy, Mono- oder Oligoglycosidreste bedeuten und wobei die Alkoxy- und Hydroxyalkoxygruppen verzweigt und unverzweigt sein und 1 bis 18 C-Atome aufweisen können und wobei an die Hydroxygruppen der genannten Reste auch Sulfat oder Phosphat gebunden sein kann,
- A: ausgewählt ist aus der Gruppe bestehend aus den Teilformeln (IA), (IB) und (IC) 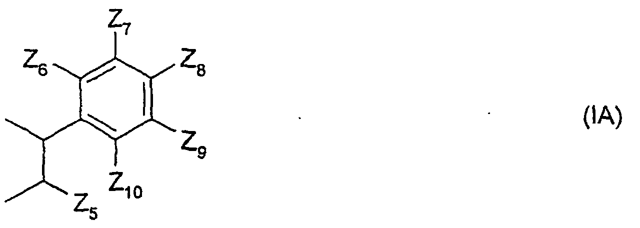 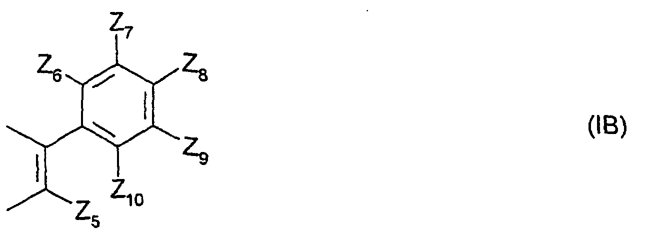 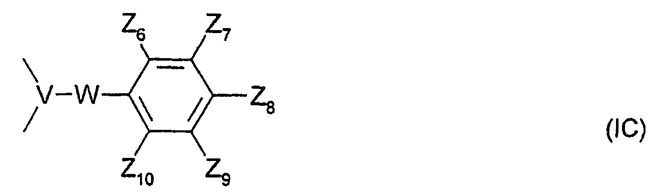
- Z₅: H, OH oder OR,
- R: einen Mono- oder Oligoglykosidrest,
- Z₆ bis Z₁₀: die Bedeutung der Reste Z₁ bis Z₄ besitzen, und 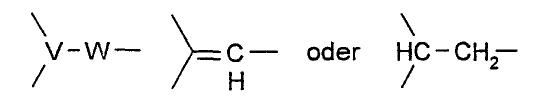
bedeutet.

Die Alkoxygruppen sind vorzugsweise linear und besitzen 1 bis 12 und vorzugsweise 1 bis 8 C-Atome. Diese Gruppen entsprechen somit den Formeln -O-(CH₂)ₘ-H, wobei m 1, 2, 3, 4, 5, 6, 7 oder 8 und insbesondere 1 bis 5 bedeutet.

Die Hydroxyalkoxygruppen sind vorzugsweise linear und besitzen 2 bis 12 und vorzugsweise 2 bis 8 C-Atome. Diese Gruppen entsprechen somit den Formeln -O-(CH₂)ₙ-OH, wobei n 2, 3, 4, 5, 6, 7 oder 8, insbesondere 2 bis 5 und außerordentlich bevorzugt 2 bedeutet.

Die Mono- und Oligoglycosidreste sind vorzugsweise aus 1 bis 3 Glycosideinheiten aufgebaut. Vorzugsweise sind diese Einheiten ausgewählt aus der Gruppe der Hexosylreste insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

In einer bevorzugten Ausführungsform besitzen
- Z₁ und Z₃ die Bedeutung H,
- Z₂ und Z₄ eine andere Bedeutung als H, insbesondere bedeuten sie OH, Methoxy, Ethoxy oder 2-Hydroxyethoxy,
- Z₅ die Bedeutung H, OH oder OR, wobei R einen Glycosidrest bedeutet, der aus 1 bis 3, vorzugsweise aus 1 oder 2, Glycosideinheiten aufgebaut ist,
- Z₆, Z₉ und Z₁₀ die Bedeutung H, und
- Z₇ und Z₈ eine andere Bedeutung als H, insbesondere bedeuten sie OH, Methoxy, Ethoxy oder 2-Hydroxyethoxy.

In einer weiteren besonders bevorzugten Ausführungsform der Erfindung, insbesondere wenn die Wasserlöslichkeit der Flavonoide und Coumaranone gesteigert werden soll, ist an die Hydroxygruppen eine Sulfat- oder Phosphatgruppe gebunden. Geeignete Gegenionen sind beispielsweise die Ionen der Alkali- oder Erdalkalimetalle, wobei diese z.B. aus Natrium oder Kalium ausgewählt sind.

Die Flavonoide sind vorzugsweise ausgewählt aus folgenden Verbindungen: 4,6,3',4'-Tetrahydroxyauron, Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten.

Unter den Flavonoiden sind insbesondere Rutin und Troxerutin bevorzugt. Ganz außerordentlich bevorzugt ist Troxerutin.

Unter den Coumaranonen ist 4,6,3',4'-Tetrahydroxybenzylcoumaranon-3 bevorzugt.

Der Anteil des einen oder der mehreren Antioxidantien in der Zubereitung beträgt vorzugsweise von 0,001 bis 5 Gew.%, besonders bevorzugt von 0,01 bis 2 Gew.% bezogen auf die gesamte Zubereitung.

Weiterhin kann in den Zubereitungen z.B. auch Anthocyanidin (Cyanidin) enthalten sein.

Die Herstellung der Zubereitung erfolgt, indem eine oder mehrere Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib gegebenenfalls mit Hilfs- und/oder Trägerstoffen in eine geeignete Zubereitungsform gebracht werden. Die Hilfs- und Trägerstoffe stammen aus der Gruppe der Trägermittel, Konservierungsstoffe und anderer üblicher Hilfsstoffe.

Die Zubereitungen auf der Grundlage einer oder mehrerer Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib werden in den für die Mund- und Rachenhygiene üblichen Applikationsformen angewendet.

Als Anwendungsform sind alle für die Mundhygiene verwendeten Applikationsformen geeignet, z.B. Lösungen, Emulsionen, Suspensionen wie z.B. Pasten, Gele, tensidhaltige Reinigungspräparate und Sprays wie z.B. Aerosolsprays und Pumpsprays. Zusätzlich zu einer oder mehreren Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib können der Zubereitung beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer, Verdickungsmittel, Feuchthaltemittel, Abrasiva und Putzkörper, Schaummittel, Verdicker, Bindemittel und Geschmacksstoffe.

Lösungen und Emulsionen können neben einer oder mehreren Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol,1,3-Butylglykol, Öle, insbesondere etherische Öle wie Pfefferminzöl, Nelkenöl, Anisöl, Fenchelöl, Salbeiöl, Glycerinfettsäureester, hydrierte Rizinusöle, Polyethylenglykole und Fettsäureester des Sorbitans, Polysorbate oder Gemische dieser Stoffe enthalten.

Die Emulsionen können in verschiedenen Formen vorliegen. So können sie z.B. eine Emulsion oder Mikroemulsion vom Typ Wasser-in-Öl (W/O), oder vom Typ Öl-in-Wasser (O/W), oder eine multiple Emulsion, beispielsweise vom Typ Wasser-in-Öl-in-Wasser (W/O/W), darstellen.

Die Zubereitungen können auch als emulgatorfreie, disperse Zubereitungen, vorliegen. Sie können beispielsweise Hydrodispersionen oder Pikkering-Emulsionen darstellen.

Suspensionen können neben einer oder mehreren Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib die üblichen Trägerstoffe wie flüssige Verdünnungsmittel und Feuchthaltemittel, z.B. Wasser, Ethanol oder Propylenglykol, Sorbitol, Glycerin, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Gele können neben einer oder mehreren Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Tensidhaltige Reinigungsprodukte können neben einer oder mehreren Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib die üblichen Trägerstoffe wie Salze von Fettalkoholsulfaten, Fettalkoholethersulfaten, Sulfobemsteinsäurehalbestem, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Sprays können neben einer oder mehreren Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sie können zusätzlich die üblichen Treibmittel, z.B. Chlorfluorkohlenwasserstoffe, Propan/Butan oder Dimethylether, enthalten.

Die Mund- und Zahnpflegemittel können z.B. in Form von Zahnpasten, flüssigen Zahncremes, Zahnpulvern, Mundwässern oder gegebenenfalls auch als Kaumasse, z.B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Die Zahnpasten oder flüssigen Zahncremes enthalten ein Poliermittel, üblicherweise in einer Menge von 5 bis 50 Gew.-%, sowie ein Feuchthaltemittel, gewöhnlich in einer Menge von 10 - 60 Gew.-%.

Als Poliermittel eignen sich alle für Zahnpasten bekannten Reibkörper wie z.B. Kieselsäuren, Aluminiumhydroxid, Aluminiumoxid, Calciumpyrophosphat, Kreide, Dicalciumphosphat-dihydrat (CaHPO₄•2H₂O), Natriumaluminiumsilikate wie z.B. Zeolith A, organische Polymere wie z.B. Polymethacrylat oder Gemische dieser Reibkörper.

Als Träger für die Zahnpasten, der die Einstellung einer geeigneten Konsistenz für die Dosierung aus Tuben, Spendebehältern oder flexiblen Flaschen ermöglicht, eignet sich beispielsweise eine Kombination aus Feuchthaltemitteln, Bindemitteln und Wasser.

Als Feuchthaltemittel können z.B. Glycerin, Sorbit, Xylit, Propylenglycole, Polyethylenglycole, insbesondere solche mit mittleren Molekulargewichten von 200-800 eingesetzt werden. Als Konsistenzregler (bzw. Bindemittel) dienen z.B. natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthan-Gum, Succinoglycan-Gum, Johannisbrotmehl, Pectine, wasserlösliche Carboxyvinylpolymere (z.B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z.B. Schichtsilikate wie z.B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol-Copolymere mit einem mittleren Molgewicht von 1000 bis 5000 oder eine Kombination der genannten Verbindungen in den Zahnpasten verwendet werden.

Auch oberflächenaktive Substanzen sind in den Zahnpasten zur Unterstützung der Reinigungswirkung und gewünschtenfalls auch zur Entwicklung von Schaum beim Zähnebürsten sowie zur Stabilisierung der Polierkörperdispersion im Träger in einer Menge von 0,1-5 Gew.-% enthalten.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z.B. Oxethylate von Fettsäuremono- und -diglyceriden, von Fettsäure-Sorbitanestern und Alkyl(oligo)-Glucoside.

Weitere übliche Zusätze zu den Mund- und Zahnpflegemitteln, insbesondere zu Zahnpasten, sind
- Süßungsmittel wie z.B. Saccharin-Natrium, Natrium-Cyclamat, Sucrose, Lactose, Maltose, Fructose,
- Aromen wie z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen,
- Pigmente wie z.B.Titandioxid
- Farbstoffe,
- Puffersubstanzen wie z.B. primäre, sekundäre oder tertiäre Alkaliphosphate oder Citronensäure/Natriumcitrat.

Die Mund- und Zahnpflegemittel, insbesondere die Zahnpasten, können neben Ectoin auch weitere Wirkstoffe enthalten.

Die Mund- und Zahnpflegemittel, insbesondere die Zahnpasten, können z.B. Anti-Karies-Wirkstoffe enthalten. Diese können beispielsweise aus organischen oder anorganischen Fluoriden ausgewählt sein, z.B. aus Natriumfluorid, Kaliumfluorid, Natriummonofluorphosphat, Zinnfluorid und Natriumfluorosilikat.

Die Mund- und Zahnpflegemittel, insbesondere die Zahnpasten, können z.B. auch Substanzen enthalten, die gegen Zahnstein wirksam sind. Derartige Substanzen können beispielsweise Chelatbildner sein wie z.B. Ethylendiamintetraessigsäure und deren Natriumsalze, Pyrophosphat-Salze wie die wasserlöslichen Dialkali- oder Tetraalkalimetallpyrophosphat-Salze, z.B. Na₄P₂O₇, K₄P₂O₇, Na₂K₂P₂O₇, Na₂H₂P₂O₇ und K₂H₂P₂O₇ oder Polyphosphat-Salze, die z.B. aus wasserlöslichen Alkalimethalltripolyphosphaten wie Natriumtripolyphosphat und Kaliumtripolyphosphat ausgewählt sein können.

Die Mund- und Zahnpflegemittel, insbesondere die Zahnpasten, können z.B. auch antimikrobielle Stoffe als Konservierungsmittel oder als Anti-Plaque-Wirkstoffe enthalten. Derartige Stoffe können z.B. ausgewählt sein aus p-Hydroxybenzoesäuremethyl-, -ethyl- oder propylester, Natriumsorbat, Natriumbenzoat, Bromchlorophen, Triclosan, Phenyl-Salicylsäureester, Biguanide z.B. Chlorhexidin, Thymol usw..

Die Mund- und Zahnpflegemittel, insbesondere die Zahnpasten, können z.B. auch wundheilende und entzündungshemmende Stoffe, z.B. Wirkstoffe gegen Zahnfleischentzündungen, enthalten. Derartige Stoffe können z.B. ausgewählt sein aus Allantoin, Azulen, Kamillenextrakten, Tocopherol, Panthenol, Bisabolol, Salbeiextrakten.

Die Mund- und Zahnpflegemittel, insbesondere die Zahnpasten, können auch Substanzen zur Erhöhung des mineralisierenden Potentials enthalten, beispielsweise calciumhaltige Substanzen wie z.B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Die Mund- und Zahnpflegemittel, insbesondere die Zahnpasten, können auch die Unempfindlichkeit der Zähne steigernde Substanzen enthalten, beispielsweise Kaliumsalze wie z.B. Kaliumnitrat, Kaliumcitrat, Kaliumchlorid, Kaliumbicarbonat und Kaliumoxalat.

In Mundwässern besteht der Träger im wesentlichen aus Wasser, Ethanol, etherischen Ölen, Emulgatoren und Lösungsvermittlern für das Ectoin und die Aromakomponenten, Geschmackskorrigentien (z.B. Süßstoff) sowie gegebenenfalls adstringierenden oder belebenden Drogenauszügen und gegebenenfalls Farbstoffen. Als weitere Wirkstoffe können z.B. noch antimikrobielle Stoffe wie Chlorhexidin oder Triclosan enthalten sein.

In einer weiteren bevorzugten Ausführungsform enthält die Zubereitung ein oder mehrere Enzyme. Das oder die Enzyme sind vorzugsweise ausgewählt aus der Gruppe bestehend aus Glucose Oxidase, Amyloglucosidase und Lactoperoxidase. Die Enzyme können z.B. eine Anti-Plaque-Wirkung besitzen. Beispielsweise kann der Zusatz des Enzyms Glucose Oxidase beim oxidativen Glucoseabbau zur Freisetzung von Wasserstoffperoxid führen, wodurch Zahnbelag, Plaque, bekämpft wird.

Enzyme besitzen häufig aber eine geringe Stabilität und sind in vitro einer Reihe destabilisierender Bedingungen ausgesetzt. Beispielsweise sind derartige Substanzen empfindlich gegenüber Veränderungen des sie umgebenden Mediums und gegenüber Temperaturschwankungen. Die Lagerung von Enzymen über einen längeren Zeitraum führt daher zu einer Abnahme der Aktivität. Besonders schwierig ist die Anwendung von Enzymen in Zubereitungen zur Mundpflege, da diese Produkte großen Temperatur- und Feuchtigkeitsschwankungen ausgesetzt sind. Dadurch kann eine Destabilisierung der Enzyme auftreten, bevor diese aus der Zubereitung an den Wirkort gelangen.

Das Problem der Abnahme der Aktivität von Enzymen tritt insbesondere auch in Zubereitungen auf, in denen sie über einen längeren Zeitraum eine möglichst hohe Aktivität besitzen müssen. Dies ist beispielsweise bei Zubereitungen mit einer gleichmäßigen Freisetzung der Enzyme über einen längeren Zeitraum, was auch als "Depot-Effekt" bezeichnet wird, der Fall.

Aus den obengenannten Gründen ist es notwendig, Enzyme in Zubereitungen zu stabilisieren. Es wurde gefunden, dass die Stabilisierung von in Zubereitungen zur Mundpflege enthaltenen Enzymen durch die ebenfalls in der Zubereitung enthaltene oder enthaltenen Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib erreicht wird.

Der Anteil der Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib in der Zubereitung beträgt vorzugsweise von 0,001 bis 50 Gew.%, besonders bevorzugt von 0,01 bis 10 Gew.% und insbesondere bevorzugt von 0,1 bis 10 Gew.% bezogen auf die gesamte Zubereitung.

Alle Verbindungen oder Komponenten, die in den Zubereitungen verwendet werden können, sind entweder bekannt und käuflich erwerbbar oder können nach bekannten Methoden synthetisiert werden.

Die folgenden Beispiele dienen zur Verdeutlichung der Erfindung und sind keinesfalls als Limitierung aufzufassen. Alle %-Angaben sind Gewichtsprozent.

Die INCI-Namen verwendeter Rohstoffe sind wie folgt (die INCI-Namen werden defintionsgemäß in Englischer Sprache angegeben):

| **Rohstoff** | **INCI-Name** |
|---|---|
| Bromchlorophen | Bromochlorophene |
| Karion F flüssig | Sorbitol, Aqua |
| Natriumbenzoat | Sodium Benzoate |
| Pfefferminzaroma | Aroma |
| Phoskadent NA 211 | Sodium Monofluorophosphate |
| Polyethylenglycol 400 DAB | PEG-8 |
| RonaCare™ CPC | N-Cetylpyridinium Chloride Monohydrate |
| RonaCare Ectoin | Ectoine |
| RonaCare NaF | Sodium Fluoride |
| RonaCare™ Olaflur | Bis(hydroxyethyl)aminopropyl-N-hydroxy- |
| | ethyl-octadecylamin-dihydrofluoride, |
| | 33 % in propanediol-1,2 |
| Saccharin-Natrium | Sodium Saccharin |
| Sident 12 | Silica |
| Sipernat 22 S | Hydrated Silica |
| Tego Betain BL 215 | Cocamidopropyl Betaine |
| Tego Betain F 50 | Cocamidopropyl Betaine |
| Tego Betain ZF | Cocamidopropyl Betaine |
| Trihydroxyethylrutin | Troxerutin |

### Beispiel 1

Aus folgenden Komponenten wird ein Mundwasserkonzentrat enthaltend Ectoin hergestellt:

| | | | | Gew.-% |
|---|---|---|---|---|
| A | RonaCare™ CPC | (Art.-Nr. 102340) | (1) | 0,2 |
| | RonaCare™ Olaflur | (Art.-Nr. 11680) | (1) | 0,5 |
| | RonaCare™ Ectoin | (Art.-Nr. 130200) | (1) | 1,0 |
| | Ethanol (96 %) | (Art.-Nr. 100971) | (1) | 20,0 |
| | Menthol | (Art.-Nr. 105995) | (1) | 0,2 |
| | Tego-Betain BL 215 | | (2) | 5,0 |
| | Glycerin (87 %) | (Art.-Nr. 104091) | (1) | 12,0 |
| | Wasser, demineralisiert | | | ad 100 |
| Herstellung: Phase A wird gerührt bis eine klare Lösung entsteht. | | | | |
| Bezugsquellen: (1) Merck KGaA (2) Goldschmidt AG | | | | |

### Beispiel 2

Aus folgenden Komponenten wird ein Zahngel enthaltend Ectoin hergestellt:

| | | | | Gew.-% |
|---|---|---|---|---|
| A | RonaCare NaF | (Art.-Nr. 106441) | (1) | 0,1 |
| | RonaCare Ectoin | (Art.-Nr. 130200) | (1) | 1,0 |
| | Natriumbenzoat | (Art.-Nr. 106290) | (1) | 0,2 |
| | Saccharin-Natrium | (Art.-Nr. 817042) | (1) | 0,2 |
| | Phoskadent | | (2) | 0,75 |
| | Karion F flüssig | (Art.-Nr. 102993) | (1) | 65,0 |
| | Wasser, demineralisiert | | | ad 100 |
| B | Bromchlorophen | (Art.-Nr. 103281) | (1) | 0,1 |
| | Pfefferminzaroma 77526- | | (3) | 1,0 |
| | 34 | | | |
| | Polyethylenglycol 400 DAB | | (3) | 3,0 |
| C | Sident 12 | | (4) | 8,5 |
| | Sipernat 22 S | | (4) | 7,5 |
| D | Perlglanzpigmente | | (1) | 0,05 |
| | Tego-Betain F 50 | | (5) | 5,0 |
| Herstellung: Die Phasen A und B werden getrennt voneinander vorgemischt und zusammengegeben. Danach wird Phase C unter Rühren langsam zugegeben und anschließend unter Vakuum auf 50 C erwärmt. Zu dem klaren Gel wird Phase D gegeben, langsam bis zur Luftfreiheit unter Vakuum gerührt und danach auf Raumtemperatur abkühlen gelassen. | | | | |
| Bezugsquellen: (1) Merck KGaA (2) Benckiser-Knapsack GmbH (3) Givaudan-Roure GmbH (4) Degussa AG (5) Th. Goldschmidt AG | | | | |

### Beispiel 3

Aus folgenden Komponenten wird ein Zahngel enthaltend Ectoin hergestellt:

| | | | Gew.-% |
|---|---|---|---|
| A | RonaCare™ NaF | (1) | 0,08 |
| | RonaCare™ Ectoin | (1) | 1,00 |
| | Trihydroxyethylrutin | (1) | 0,50 |
| | Karion F flüssig | (1) | 62,13 |
| | Natriumbenzoat | (1) | 0,20 |
| | Natriumsaccharinat | | 0,20 |
| | Wasser, demineralisiert | | 9,00 |
| B | RonaCare™ Olaflur | (1) | 1,50 |
| | Bromchlorophen | (1) | 0,10 |
| | Aroma 35049 | (2) | 1,00 |
| C | Polyethylenglycol 400 | (1) | 3,00 |
| | Tego Betain ZF | (3) | 5,00 |
| | Sicomet Patent Blau (E131), 0,1 % in | (4) | 0,80 |
| | Wasser | | ad 100 |
| D | Sident 12 | (5) | 9,50 |
| | Sipernat 22 S | (5) | 7,50 |
| Herstellung: Die Phasen A und B werden getrennt voneinander vorgemischt. Phase C wird auf 50°C erhitzt, die Phasen A und B in die Phase C eingerührt und anschließend wird unter Vakuum vermischt. Nach langsamer Zugabe von Phase D wird unter Vakuum homogenisiert. Es wird weiter unter Vakuum gerührt bis das Gel klar ist. | | | |
| Bezugsquellen: (1) Merck KGaA (2) Orissa Drebing GmbH (3) Th. Goldschmidt AG (4) BASF AG (5) Degussa AG. | | | |

## Patentansprüche

1. Verwendung einer oder mehrerer Verbindungen ausgewählt aus den Verbindungen der Formeln Ia und Ib 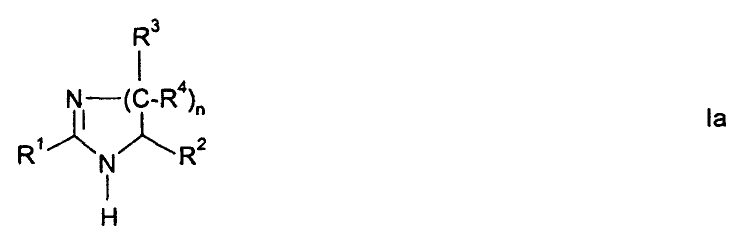 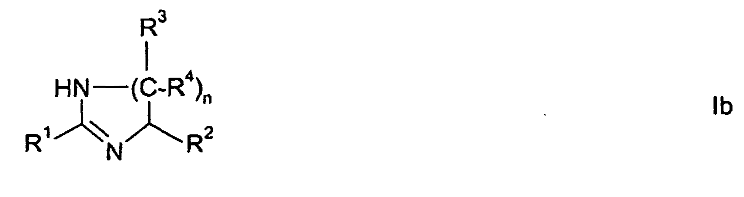 den physiologisch verträglichen Salzen der Verbindungen der Formeln Ia und Ib, und den stereoisomeren Formen der Verbindungen der Formeln Ia und Ib, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten, zur Herstellung einer Mundpflegezubereitung zum Schutz und zur Pflege der residenten Mundflora.

2. Verwendung nach Anspruch 1 zum prophylaktischen Schutz der Zähne gegen eine Schädigung des Zahnschmelzes.

3. Verwendung nach einem oder mehreren der Ansprüche 1 bis 2 zum prophylaktischen Schutz der Mund- und Rachenschleimhaut.

4. Verwendung nach einem oder mehreren der Ansprüche 1 bis 3 **dadurch gekennzeichnet, dass** man eine oder mehrere Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib zur Anwendung in Form einer Lösung, einer Emulsion, einer Suspension, einer Paste, eines Gels, eines tensidhaltigen Reinigungspräparates, eines Sprays, einer Creme, eines Pulvers, eines Mundwassers oder einer Kaumasse einsetzt.

5. Verwendung nach einem oder mehreren der Ansprüche 1 bis 4, **dadurch gekennzeichnet, dass** der Anteil der Verbindungen ausgewählt aus den Verbindungen der Formeln Ia und Ib, den physiologisch verträglichen Salzen der Verbindungen der Formeln Ia und Ib, und den stereoisomeren Formen der Verbindungen der Formeln Ia und Ib von 0,001 bis 50 Gew.% bezogen auf die gesamte Zubereitung beträgt.

6. Verwendung nach einem oder mehreren der Ansprüche 1 bis 5, **dadurch gekennzeichnet, dass** die Verbindungen der Formeln Ia und Ib ausgewählt sind aus den Verbindungen (S)-1,4,5,6-Tetrahydro-2-methyl-4-pyrimidincarbonsäure und (S,S)-1,4,5,6-Tetrahydro-5-hydroxy-2-methyl-4-pyrimidincarbonsäure.

7. Verwendung nach einem oder mehreren der Ansprüche 1 bis 6, **dadurch gekennzeichnet, dass** die Zubereitung ein oder mehrere Antioxidantien enthält.

8. Verwendung nach einem oder mehreren der Ansprüche 1 bis 7, **dadurch gekennzeichnet, dass** die Zubereitung ein oder mehrere Enzyme enthält.

## Claims

1. Use of one or more compounds selected from the compounds of the formulae Ia and Ib 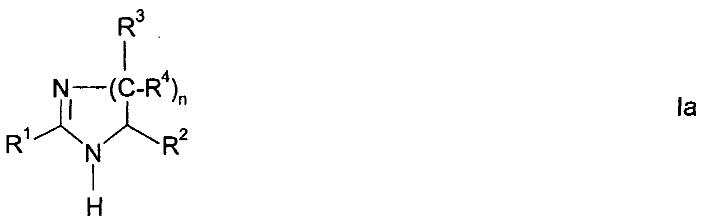 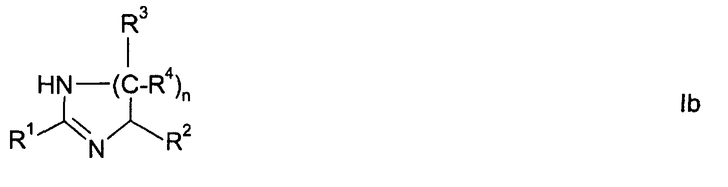 the physiologically tolerated salts of the compounds of the formulae Ia and Ib, and the stereoisomeric forms of the compounds of the formulae Ia and Ib, where
R¹ denotes H or alkyl,
R² denotes H, COOH, COO-alkyl or CO-NH-R⁵,
R³ and R⁴ each, independently of one another, denote H or OH,
n denotes 1, 2 or 3,
alkyl denotes an alkyl radical having 1 to 4 carbon atoms, and
R⁵ denotes H, alkyl, an amino acid radical, dipeptide radical or tripeptide radical,
for the preparation of an oral care composition for the protection and care of the resident oral flora.

2. Use according to Claim 1 for the prophylactic protection of teeth against damage to the dental enamel.

3. Use according to one or more of Claims 1 to 2 for the prophylactic protection of the oral and pharyngeal mucous membrane.

4. Use according to one or more of Claims 1 to 3, **characterised in that** use is made of one or more compounds selected from the compounds of the formulae Ia and Ib, the physiologically tolerated salts of the compounds of the formulae Ia and Ib and the stereoisomeric forms of the compounds of the formulae Ia and Ib for use in the form of a solution, an emulsion, a suspension, a paste, a gel, a surfactant-containing cleansing preparation, a spray, a cream, a powder, a mouthwash or a chewing composition.

5. Use according to one or more of Claims 1 to 4, **characterised in that** the proportion of the compounds selected from the compounds of the formulae Ia and Ib, the physiologically tolerated salts of the compounds of the formulae Ia and Ib, and the stereoisomeric forms of the compounds of the formulae Ia and Ib is 0.001 to 50% by weight, based on the composition as a whole.

6. Use according to one or more of Claims 1 to 5, **characterised in that** the compounds of the formulae Ia and Ib are selected from the compounds (S)-1,4,5,6-tetrahydro-2-methyl-4-pyrimidinecarboxylic acid and (S,S)-1,4,5,6-tetrahydro-5-hydroxy-2-methyl-4-pyrimidine-carboxylic acid.

7. Use according to one or more of Claims 1 to 6, **characterised in that** the composition comprises one or more antioxidants.

8. Use according to one or more of Claims 1 to 7, **characterised in that** the composition comprises one or more enzymes.

## Revendications

1. Utilisation d'un ou de plusieurs composés choisis parmi les composés des formules Ia et Ib 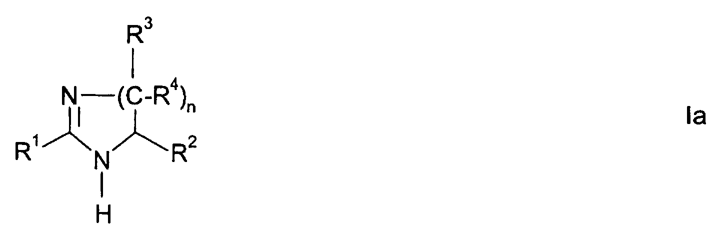 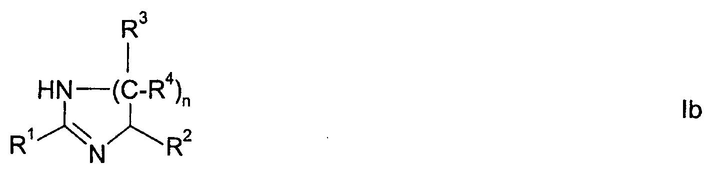 des sels tolérés physiologiquement des composés des formules Ia et Ib, et des formes stéréoisomériques des composés des formules Ia et Ib, dans lesquelles
R¹ représente H ou alkyle,
R² représente H, COOH, COO-alkyle ou CO-NH-R⁵,
R³ et R⁴ représentent, chacun indépendamment l'un de l'autre, H ou OH,
n représente 1, 2 ou 3,
alkyl représente un radical alkyle comportant de 1 à 4 atomes de carbone, et
R⁵ représente H, alkyle, un radical acide aminé, un radical dipeptide ou un radical tripeptide,
pour la préparation d'une composition de soin buccal pour la protection et le soin de la flore buccale résidente.

2. Utilisation selon la revendication 1 pour la protection prophylactique des dents vis-à-vis d'un endommagement de l'émail dentaire.

3. Utilisation selon une ou plusieurs des revendications 1 et 2 pour la protection prophylactique de la membrane de la muqueuse buccale et pharyngienne.

4. Utilisation selon une ou plusieurs des revendications 1 à 3, **caractérisée en ce que** l'on utilise un ou plusieurs composés choisis parmi les composés des formules Ia et Ib, les sels tolérés physiologiquement des composés des formules Ia et Ib et les formes stéréoisomériques des composés des formules Ia et Ib pour une utilisation sous la forme d'une solution, d'une émulsion, d'une suspension, d'une pâte, d'un gel, d'une préparation de nettoyage/démaquillage contenant un agent tensio-actif, d'un spray, d'une crème, d'une poudre, d'un bain de bouche ou d'une composition à mâcher.

5. Utilisation selon une ou plusieurs des revendications 1 à 4, **caractérisée en ce que** la proportion des composés choisis parmi les composés des formules Ia et Ib, des sels tolérés physiologiquement des composés des formules Ia et Ib, et des formes stéréoisomériques des composés des formules Ia et Ib est de 0,001 % à 50% en poids, sur la base de la composition considérée dans sa globalité.

6. Utilisation selon une ou plusieurs des revendications 1 à 5, **caractérisée en ce que** les composés des formules Ia et Ib sont choisis parmi les composés acide (S)-1,4,5,6-tétrahydro-2-méthyl-4-pyrimidine-carboxylique et acide (S,S)-1,4,5,6-tétrahydro-5-hydroxy-2-méthyl-4-pyrimidinecarboxylique.

7. Utilisation selon une ou plusieurs des revendications 1 à 6, **caractérisée en ce que** la composition comprend un ou plusieurs antioxydants.

8. Utilisation selon une ou plusieurs des revendications 1 à 7, **caractérisée en ce que** la composition comprend un ou plusieurs enzymes.
